# EUROPEAN PATENT APPLICATION

(11) **EP 3 170 480 A1**
(43) Date of publication of application: **24.05.2017**
(21) Application number: 15195094.6
(22) Date of filing: 18.11.2015
(51) Int. Cl.: A61F 13/00

(54) **APPARATUS AND PROCESS FOR RECYCLING HEATED GAS**

(71) Applicant: The Procter & Gamble Company, Cincinnati, OH 45202 (US)
(72) Inventor: JACKELS, Hans Adolf, 53881 Euskirchen (DE)
(74) Representative: Mather, Peter Geoffrey

(57) **Abstract**

The present invention relates to a recycling apparatus for recycling a heated gas, preferably heated air, the apparatus comprising a Venturi nozzle.

The present invention also relates to process for recycling a heated gas, the process comprising regulating the flow of gas by a Venturi nozzle.

## Description

### Field of the Invention

The present invention relates to an apparatus and a process for recycling heated gas, preferably heated air.

### Background

Absorbent articles for personal hygiene, such as disposable diapers for infants, training pants for toddlers, adult incontinence undergarments, and/or sanitary napkins are designed to absorb and contain bodily exudates. The application of heated gas may be useful in the process of manufacturing absorbent articles, for example hot gas may be used to heat a nonwoven web to facilitate the deformation or bulking of the web, or hot gas may be used to partially melt portions of a nonwoven web to join together two components, or two parts of a single component, by hot seaming including hot air seaming. Heated gas may also be useful in manufacturing processes for a host of other products.

EP-A-1 403 413, published on March 31st 2004, discloses a method for restoring bulkiness of nonwoven fabric by blowing hot air heated at a prescribed temperature toward the conveyer belt carrying a nonwoven fabric and sucking the hot air away from the opposite side of the fabric-carrying belt.

Energy is required to heat up the hot gas to the operating temperature and it is preferable to recirculate the hot gas to improve energy efficiency, target temperature reponse/ramp time and air costs.

It is desirable to provide an improved hot gas recirculation system with improved control over the regulation of gas flow within the system.

### Summary of the Invention

The present invention relates to a recycling apparatus for recycling a heated gas, preferably heated air, the apparatus comprising:
a) a gas injection nozzle to inject gas into the apparatus;
b) a blower to propel the gas through the apparatus;
c) a heater to heat the gas;
d) a heating tool to apply the heated gas to a workpiece;
e) a recycling duct to return heated gas to be mixed with gas injected by the gas injection nozzle;
and wherein the recycling apparatus further comprises a Venturi nozzle.

In another embodiment, the present invention relates to a process for recycling a heated gas, the process comprising:
a) injecting a gas into the apparatus through a gas injection nozzle;
b) propelling the gas through the apparatus by a blower;
c) heating the gas in a heater
d) applying the heated gas to a workpiece at a heating tool;
e) recycling the heated gas through a recycling duct to the gas injection nozzle;
and regulating the flow of gas through the recycling process by a Venturi nozzle.

### Brief Description of the Drawings

Figure 1 is a schematic representation of a recycling apparatus according to the present invention.
Figures 2a and 2b are schematic representations showing enlarged views of part of the recycling apparatus. Figures 2a and 2b are alternative embodiments of the present invention showing a heated roll and workpiece.

### Detailed Description of the Invention

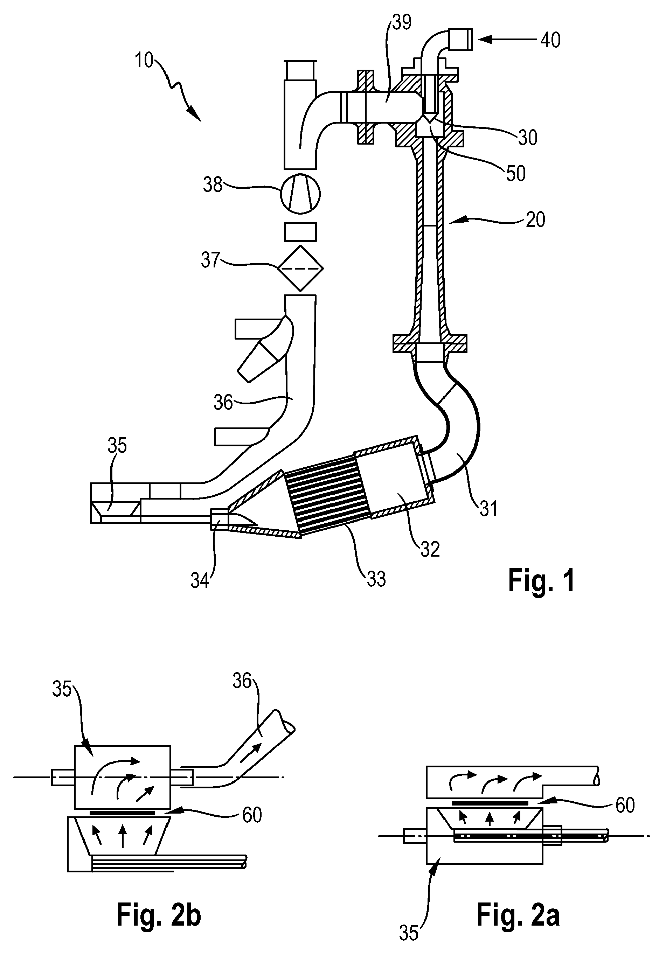

Figure 1 is a schematic drawing showing a hot gas recycling apparatus of the present invention 10. Whilst the gasses employed in the recycling apparatus may be any suitable gas, including air, steam, for example, in the following description the gas will be referred to as air, although the invention is not limited to air.

Compressed air enters the apparatus through the compressed air inlet 40 and a gas injection nozzle 30. At the outlet of the gas injection gun 30, the compressed air is mixed with recycled hot air in the mixing chamber 50. The volumetric flow and pressure of these gasses in the mixing chamber 50 is controlled by a Venturi nozzle 20.

The air exits the Venturi nozzle 20 through a first air duct 31. Blower 32 propels the air through heater 33 to achieve the desired temperate and pressure. The hot air then passes via a hot air distributor 34 to heating tool 35 to heat item to be treated, referred to hereafter as workpiece 60.

After heating the workpiece 60, the air is channeled via a second air duct 36 to a filter 37 so that any foreign matter, such as fibrous material, can be extracted from the air flow. A blower 38 returns the air through a recycling duct 39 to the mixing chamber 50, and the process is repeated.

In a preferred embodiment of the present invention, shown schematically in Figure 2a, the workpiece 60 is a web material, most preferably a nonwoven web. The heating tool may comprise a belt, such as a continuous foraminous belt, but preferably the heating tool 35 is a roll, most preferably a perforated roll to allow the hot air to pass from the interior of the roll through the perforations and through the workpiece 60. Alternatively, as shown in Figure 2b, the hot air may be channeled from the outside of the roll, through the workpiece 60, then through the perforations and into the interior of the roll.

### Venturi

The Venturi effect is the reduction in fluid pressure that results when a fluid flows through a constricted section of pipe 20. In the present invention the Venturi nozzle 20 provides accurate flow metering of the gas (air, for example). The reduction in pressure serves to regulate the flow of incoming gases from the recycling duct 38 and from the gas injection nozzle 50. The Venturi nozzle 20 regulates the flow independently of the temperature of the gasses, whilst the overall pressure loss across the nozzle is low resulting in overall energy savings. By comparison, an orifice plate would result in higher overall pressure loss and greater energy loss. The Venturi nozzle 20 requires minimal upstream straight length in comparison to an orifice plate and the nozzle is inherently robust with no maintenance and inspection required.

### Example

The following example compares overall energy requirements of a system with no recycling compared to a recycling system comprising a Venturi nozzle according to the present invention.

No Recycling (Comparative):

| | |
|---|---|
| Temperature of Air at Workpiece 60 | 100°C |
| Volumetric Flow Rate of Air | 273.2 m³/hr |
| Required Air Mass Stream | 258.6 kg/hr |
| Total Power for Heating Air | 5.8 kW |

With Recycling:

| | |
|---|---|
| Temperature of Air at Workpiece 60 | 100°C |
| Flow Rate of Compressed Air through Nozzle 30 | 52.5 kg/hr |
| Power of Heater 33 for Compressed Air | 1.18 kW |
| Power of Heater 33 for Recycled Air | 2.31 kW |
| Total Power for Heating Air | 3.49 kW |

The total power required for heating air in the recycling apparatus, 3.49 kW, is 40% lower than the total power required for heating air with no recycling, 5.8 kW.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A recycling apparatus (10) for recycling a heated gas, the apparatus (10) comprising:
a) a gas injection nozzle (30) to inject gas into the apparatus (10);
b) a blower (32, 37) to propel the gas through the apparatus (10);
c) a heater (33) to heat the gas;
d) a heating tool (35) to apply the heated gas to a workpiece (60);
e) a recycling duct (39) to return the heated gas to be mixed with gas injected by the gas injection nozzle (30);
**characterized in that** the recycling apparatus (10) further comprises a Venturi nozzle (20).

2. The recycling apparatus of Claim 1 wherein the Venturi nozzle (20) is positioned between the nozzle (30) and the heater (33).

3. The recycling apparatus of Claim 1 wherein the Venturi nozzle (20) is positioned between the nozzle (30) and the blower (32).

4. The recycling apparatus of any of Claims 1 to 3 wherein the heated gas is heated air.

5. The recycling apparatus of Claim 4 wherein the gas injection nozzle (30) injects compressed air into the apparatus (10).

6. The recycling apparatus of any of the previous Claims wherein the workpiece (60) is a nonwoven web.

7. The recycling apparatus of any of the previous Claims wherein the heating tool (35) is a roll, preferably a perforated roll through which the heated gas passes.

8. A process for recycling a heated gas, the process comprising:
a) injecting a gas into the apparatus (10) through a gas injection nozzle (30);
b) propelling the gas through the apparatus (10) by a blower (32, 37);
c) heating the gas in a heater (33)
d) applying the heated gas to a workpiece (60) at a heating tool (35);
e) recycling the heated gas through a recycling duct (39) to the gas injection nozzle (30)
**characterized in that** the process further comprises the step of regulating the flow of gas by a Venturi nozzle (20).

9. The process according to Claim 8 wherein the Venturi nozzle (20) is positioned between the injection step and the heating step.

10. The process according to Claim 8 wherein the Venturi nozzle (20) is positioned between the injection step and the blowing step.

11. The process according to any of Claims 8 to 10 wherein the heated gas is heated air.

12. The process according to Claim 11 wherein compressed air is injected through the gas injection nozzle (30).

13. The process according to any of Claims 8 to 12 wherein the workpiece (60) is a nonwoven web.

14. The process according to any of the Claims 8 to 13 wherein the heated gas passes through across the heating tool (35), wherein the heating tool (35) is a roll.
